# EUROPEAN PATENT APPLICATION

(11) **EP 4 431 916 A1**
(43) Date of publication of application: **18.09.2024**
(21) Application number: 21963628.9
(22) Date of filing: 12.11.2021
(51) Int. Cl.: G01N 21/64, C12M 1/34

(54) **METHOD FOR IMPROVING SEQUENCING RESOLUTION, AND SEQUENCING APPARATUS AND SYSTEM**

(71) Applicant: BGI Shenzhen, Shenzhen, Guangdong 518083 (CN)
(72) Inventor: SU, Zeyu, Shenzhen, Guangdong 518083 (CN); LIANG, Yuanqing, Shenzhen, Guangdong 518083 (CN); SHEN, Mengzhe, Shenzhen, Guangdong 518083 (CN); NI, Jielei, Shenzhen, Guangdong 518083 (CN)
(74) Representative: Ashton, Gareth Mark
(86) International application number: PCT/CN2021/130332
(87) International publication number: WO 2023/082179

(57) **Abstract**

A method for improving sequencing resolution, and a sequencing apparatus and system. The method comprises: performing, by using at least one type of excitation light, irradiation excitation on a sample to be tested that contains fluorescent dye, so as to generate a fluorescence signal, wherein in the excitation light, the maximum wavelength is less than the wavelength of red light; and transmitting the generated fluorescence signal by means of an optical path, and then collecting and imaging same.

## Description

### TECHNICAL FIELD

The present disclosure relates to the field of biotechnologies, and particularly to a method for increasing a sequencing resolution, and a sequencing device and system.

### BACKGROUND

DNA sequencing technology is one of the most commonly used technical means in researches related to molecular biology, and also promotes the rapid development of the art at the same time. Human genome project, transcriptome analysis, microbial genome resequencing, single nucleotide polymorphisms (SNP) analysis, and the like also greatly promote the research and development in other biological fields.

The replacement of each generation of sequencing technology marks a new breakthrough in the technical fields of gene chip, data analysis, surface chemistry and bioengineering in biology, which is mainly manifested in the reduction of sequencing cost and the improvement of sequencing efficiency, thus realizing sequencing development in directions of high throughput, low cost, high security and commercialization.

Although a first generation of DNA sequencing technology has helped people to complete a lot of sequencing works with a sequencing reading length of 1,000 bp and a high accuracy of 99.99%, the sequencing technology has shortcomings such as a slow testing speed, a high cost and a low throughput, so that the sequencing technology cannot be popularized and commercialized.

In 2005, Roche Company released a 454 sequencing system, which marked that the sequencing technology entered a second generation of DNA sequencing technology. The second generation of DNA sequencing technology, also known as a high-throughput sequencing (HTS) technology, performs rapid sequencing and analysis on hundreds of thousands to millions of DNA molecules in hundreds or thousands of samples at one time with a low cost and an accuracy over 99%, with representative technologies including 454 from Roche Company, Solexa from Illumina Company and SOLID from ABI Company.

The second generation of DNA sequencing technology is a core of epoch-making change of the first generation of sequencing technology. At present, a sequencing system mainly based on the second generation of DNA sequencing technology identifies and distinguishes 4 basic groups (A, T/U, C and G) by a method of light signal detection, which is certainly realized on the premise of fluorescently labeling the basic groups.

In common sequencing technologies, 4 fluorescent dyes are generally used to label 4 basic groups respectively. Meanwhile, there is also a testing means of identifying and distinguishing 4 basic groups with 2 fluorescent dyes, for example, a sequencing method based on dual fluorescent dyes is used in a NextSeq sequencing system and a Mini-Seq sequencing system developed by Illumina Company. Whether 4 fluorescent dyes or 2 fluorescent dyes are used for dyeing, an excitation light source is inevitably matched.

At present, red and green excitation light sources are mostly used in sequencing technologies on the market. According to Rayleigh criterion, a resolution of an optical system is x=0.61 λ/NA. Taking a red and green excitation light sequencing technology of Illumina and MGI-DNBSEQTM as an example, a fluorescence center wavelength excited by the red light is about 720 nm, and an NA of a lens is 0.8, thus it can be known that a theoretical resolution is about 550 nm. Because the NA of the lens is affected by a production process and very difficult to improve, it is very difficult for the red and green excitation light sequencing technology to make a breakthrough in resolution, thus limiting a sequencing throughput and a sequencing quality to a certain extent.

In order to improve the sequencing quality, or to achieve the purpose of increasing the sequencing throughput by providing a density of DNA binding sites on a chip surface, how to increase the resolution of the sequencing technology will become a technical problem to be urgently solved by those skilled in the art.

In view of this, the present disclosure is specifically proposed.

### SUMMARY

The present disclosure provides a method for increasing a sequencing resolution, so as to solve the technical problem that it is actually very difficult for a red and green excitation light sequencing technology to make a breakthrough in resolution, thus limiting a sequencing throughput and a sequencing quality to a certain extent in an existing sequencing technology.

In another aspect, the present disclosure further provides a sequencing device for implementing the method for increasing the sequencing resolution, so that the breakthrough in sequencing resolution is achieved through the combination of the sequencing device and the method. In yet another aspect, the present disclosure further provides a sequencing system for application.

A method for increasing a sequencing resolution includes the following steps of:
carrying out irradiation and excitation on a sample to be tested containing a fluorescent dye with at least one type of excitation light to generate a fluorescence signal, where a maximum wavelength in the excitation light is smaller than a wavelength of red light; and
transmitting the generated fluorescence signal through an optical path, and collecting and imaging the signal.

In the method, a core lies in specific selection of the excitation light and the wavelength, and at least one type of excitation light irradiates the sample to be tested containing the fluorescent dye to generate the fluorescence signal; and the fluorescence signal is transmitted through the optical path, and then collected and imaged, so as to achieve the purpose of sequencing. Because there is the excitation light with the maximum wavelength smaller than the wavelength of the red light, according to Rayleigh criterion, the resolution is x=0.61 X/NA, and in the present disclosure, when a material cost is not increased, excitation light with a shorter wavelength is used instead of the original red and green light, which increases the detection resolution, thus further increasing a sequencing throughput. The present disclosure solves the technical problem that it is very difficult for a red and green excitation light sequencing technology to make a breakthrough in resolution, thus limiting a sequencing throughput and a sequencing quality to a certain extent in an existing sequencing technology.

It should be understood that, in the present disclosure, "a sample to be tested containing a fluorescent dye" may exist in various forms in practical application scenarios, as long as it achieves the purpose of labeling the sample to be tested having specific physical and chemical characteristics with the fluorescent dye. More specifically, "a sample to be tested containing a fluorescent dye" may be a nucleic acid sequence for binding the fluorescent dye by some binding enzymes.

Preferably, the excitation light takes blue light as first excitation light and takes green light as second excitation light.

It should be further described that, in some cases, an optical spectrum of the second excitation light may also cover a yellow light, so that the second excitation light is most preferably the green light, but an embodiment containing the yellow light is not excluded.

Preferably, a wavelength of the first excitation light ranges from 440 nm to 500 and a wavelength of the second excitation light ranges from 500 nm to 600 nm.

More preferably, a wavelength of the first excitation light ranges from 470 nm to 490 and a wavelength of the second excitation light ranges from 550 nm to 580 nm.

Preferably, the two types of excitation light are coupled through the optical fibers and share one optical fiber port for output and transmission to the sample to be tested, and a type of the optical fiber port is one of FC/PC or FC/APC or SMA.

The first excitation light and the second excitation light are used, so that at least two types of fluorescence signals may be excited, and the first excitation light is the blue light with the wavelength ranging from 440 nm to 500 nm, and the wavelength is smaller than wavelengths of traditional red and green light, thus improving detection efficiency; and preferably, the FC/APC interface is used, and the FC/APC interface can reduce a return loss, thus further guaranteeing a detection result.

Preferably, uniformities of light spots of the two types of excitation light are both greater than 85%; lengths of optical fibers of the two types of excitation light are both greater than 2,000 mm; and output power of the optical fibers is greater than or equal to 2,000 mw.

The parameters of the light sources above are not the only specific limitations as a preferred way to increase the sequencing resolution.

Preferably, the fluorescent dye includes a first fluorescent dye excited by the first excitation light and a second fluorescent dye excited by the second excitation light;
the first fluorescent dye includes: one or more of Alexa Fluor 488, iF488, ATTO488, DyLight488, Hilyte488, FAM488 or FITC488; and
the second fluorescent dye includes: one or more of CY3.5, AF568 or ROX.

The preferred specific fluorescent dyes above correspond to the wavelengths of the two types of excitation light, so as to improve excitation efficiency as much as possible. In addition, in the present disclosure, the first fluorescent dye and the second fluorescent dye above may both be directly purchased.

Preferably, a dosage ratio of the first fluorescent dye to the second fluorescent dye is 3: 1 to 4: 1.

The first fluorescent dye and the second fluorescent dye in the dosage ratio above are more prone to meet a sequencing requirement with a high standard, and in practical application, a dosage unit of the two fluorescent dyes is preferably µmol.

Preferably, the transmitting through the optical path further includes the step of respectively guiding the fluorescence signal and a non-fluorescence signal to different optical paths by using two types of dichroscopes; where a transmission window of one type of dichroscope is 500 nm to 550 nm and/or 580 nm to 650 nm; and a transmission window of the other type of dichroscope is at least 800 nm.

The dichroscopes may distinguish light with different wavelengths in forms of reflection and transmission, and a reflectivity and a transmittance to light with a specific wavelength range are both greater than 95%. It should be understood that specific setting scenes of the two types of dichroscopes are selected according to effects realized in the optical path, and setting positions of the two types of dichroscopes are not unique.

Preferably, the transmitting the fluorescence signal through the optical path further includes the operation of making the signal pass through a filtering component with a transmission window of 483 nm to 550 nm or 580 nm to 700 nm.

Preferably, the sample to be tested includes DNA and is arranged on a substrate the substrate includes one of a silicon substrate, glass or sapphire.

A sequencing device for implementing the method above is provided, where the sequencing device is provided with an excitation light source assembly capable of emitting the excitation light with the maximum wavelength smaller than the wavelength of the red light, an optical path assembly at least including a dichroscope and an objective lens, and an imaging assembly; the dichroscope is arranged on an optical path formed after the excitation light is emitted by the excitation light source assembly for reflection, transmission or distinguishment of the excitation light and/or the fluorescence signal generated after the sample to be tested is excited by the excitation light; and the objective lens is arranged on an optical path through which the fluorescence signal enters the dichroscope; and the imaging assembly is arranged on an optical path formed after the fluorescence signal is distinguished for collecting and imaging different fluorescence signals.

In the present disclosure, after the excitation light passes through the optical path assembly of the dichroscope and the objective lens, the sample to be tested is excited to generate the fluorescence signal, and the fluorescence signal is subjected to a series of reflections, transmissions or distinguishments in the optical path composed of the optical path assembly of the dichroscope and the objective lens, and is finally collected and imaged by the imaging assembly arranged at a tail end of the optical path, so as to achieve the purpose of detection.

The sequencing device of the present disclosure meets the method for increasing the sequencing resolution above through a hardware carrier, compared with traditional red and green excitation light, the wavelength of the excitation light emitted by the light source assembly of the device is shorter, and according to Rayleigh criterion (a resolution is x=0.61 λ/NA), on the premise that an NA is unchanged, the present disclosure breaks through a theoretical resolution (550 nm) in the existing technology, improves a sequencing quality or a sequencing throughput, and reduces a sequencing cost to a certain extent.

Preferably, the excitation light source assembly includes two excitation light sources, where one excitation light source excites the blue light as the first excitation light, and the other excitation light source excites the green light as the second excitation light; and
the two excitation light sources are coupled through the optical fibers, and the two types of excitation light share one optical fiber port for output.

In specific application, the excitation light source assembly generally needs to emit at least two types of excitation light with different wavelengths, both of which are smaller than the wavelength of the red light, so as to respectively excite the fluorescent dyes in the sample to be tested and generate two fluorescence signals. Certainly, in the present disclosure, the distinguishing function of the dichroscope not only refers to distinguishing the fluorescence signal from other light, but also is manifested in distinguishing two different fluorescence signals and then imaging the signals by the imaging assembly when there are two types of excitation light.

As mentioned above, the wavelength of the blue light (first excitation light) ranges from 440 nm to 500 nm, and the wavelength preferably ranges from 470 nm to 490 nm, with optional components including a semiconductor laser device of 470 nm, a semiconductor pumped solid-state (DPSS) laser device of 473 nm or a semiconductor laser device of 488 nm; and
the wavelength of the other light source (green light) ranges from 500 nm to 600 nm, and the wavelength preferably ranges from 550 nm to 580 nm, with for example a solid-state laser device of 556 nm, a solid-state semiconductor (DPSS) laser device of 561 nm or a solid-state laser device of 577 nm. In addition, the light source may also be an LED.

The two excitation light sources are coupled through the optical fibers, and the two types of excitation light share one optical fiber port for output, so that the whole device is more integrated and convenient to control.

Preferably, the optical path assembly further includes an automatic focusing component; the automatic focusing component emits probe light with a wavelength different from the wavelength of the fluorescence signal; and the probe light is transmitted through the dichroscope, the objective lens and the sample to be tested and returns in the same path the automatic focusing component drives the objective lens to realize focusing according to a feedback signal collected after returning.

The probe light with the wavelength different from the wavelength of the fluorescence signal emitted by the automatic focusing component is generally infrared light, with a waveband of 800 nm to 900 nm, and in a preferred embodiment, the waveband is 830 nm to 860 nm. The probe light emitted by the automatic focusing component may pass through the dichroscope and the objective lens in sequence and then is irradiated to the sample to be tested, and then returns to the automatic focusing component in the original path, and the automatic focusing component drives the objective lens to move to a focal plane position according to the collected feedback signal to realize the focusing function.

Preferably, the dichroscope includes a second dichroscope, a first dichroscope and a third dichroscope arranged from top to bottom, where the third dichroscope and the second dichroscope are communicated through an optical path and arranged adjacently.

The dichroscope distinguishes excitation light, probe light or fluorescence signals with different wavelengths through reflection/transmission; and because the second dichroscope and the first dichroscope are arranged from top to bottom, the excitation light enters the first dichroscope first and then is reflected, and passes through the objective lens and then reaches the sample to be tested, thus prompting the generation of the fluorescence signal. The fluorescence signal passes through the objective lens, penetrates through the first dichroscope, and then is reflected by the second dichroscope arranged above the first dichroscope, and because the third dichroscope and the second dichroscope are communicated through the optical path and arrange adjacently, the fluorescence signal is reflected to the third dichroscope, distinguished, and collected and imaged by the imaging assembly.

Preferably, the imaging assembly includes a cylindrical lens and a camera which are arranged on different output optical paths of the third dichroscope; and a reflecting component is also arranged between the cylindrical lens and the camera.

When two types of excitation light are used, two fluorescence signals will be generated, which are respectively output by different optical paths after being distinguished by the third dichroscope, and at the moment, the collection and imaging of the fluorescence signals may be respectively realized by the cylindrical lens, the reflecting component and the camera arranged on the optical paths.

Preferably, a collimating lens is arranged on an optical path between the excitation light source assembly and the first dichroscope; a free end of the collimating lens is used for accessing the excitation light emitted by the excitation light source assembly; and the other end of the collimating lens extends inside the first dichroscope.

The collimating lens is mainly used as an optical path carrier of the excitation light through a special positional relationship, so that the excitation light enters the first dichroscope from the excitation light source assembly, and meanwhile, the first dichroscope may reflect the excitation light to the objective lens to reach the sample to be tested, so as to excite the fluorescence signal.

Preferably, the sequencing device further includes a mounting base, where the dichroscope and the imaging assembly are arranged on the mounting base;
and/or; when the optical path assembly includes the automatic focusing component, the focusing components is arranged on the mounting base; and
one end of the objective lens penetrates through the mounting base and is used for being opposite to the sample to be tested, and the other end of the objective lens is communicated with the first dichroscope and/or the second dichroscope.

The mounting base is used as a bearing platform for the dichroscope (the first dichroscope, the second dichroscope and the third dichroscope), the imaging assembly, the automatic focusing component, the collimating lenses and other components, so as to mount and fix the components and integrate the components.

It should be understood that the mounting base plays a role in fixing and mounting the above components on one hand, and is also a carrier for connecting and fixing the whole device with other functional parts of the sequencing system, so that mounting holes or mounting cavities in various forms may be arranged on the mounting base according to actual requirements.

Preferably, a data interface for data exchange is preset on the camera;
and/or; when the optical path assembly includes the focusing component, a data interface for data exchange is preset on the automatic focusing component.

The data interface is a guarantee for direct connection between the automatic focusing component or the camera and other functional modules (such as a power supply and a control system). It is easy to understand that actions of data exchange with other functional modules of the automatic focusing component or the camera may also be implemented by Bluetooth or other wireless connection ways.

Preferably, the sequencing device further includes the filtering component, where the filtering component is arranged in an optical path in front of or behind the cylindrical lens; and in a preferred scheme, the filtering component is arranged between the third dichroscope and the cylindrical lens.

Preferably, the objective lens is provided with a plurality of optical lenses, and the objective lens is a dry or immersive objective lens, with a magnification factor not less than 16 times.

The filtering component (for shielding the excitation light reflected by the sample), the plurality of optical lenses and the magnification factor are all intended to guarantee detection efficiency as much as possible from hardware.

A sequencing system includes a sample to be tested and the sequencing device above, where,
the sample to be tested includes a DNA sequence and a fluorescent dye, and is opposite to the objective lens; and
when the fluorescent dye is excited by the excitation light emitted by the excitation light source assembly, the fluorescence signal is generated and enters the optical path of the dichroscope through the objective lens.

Preferably, the sequencing system further includes a control component, where the control component is connected with one or more assemblies of the sequencing device for data exchange, control or display.

To sum up, according to the method for increasing the sequencing resolution, and the sequencing device and system provided by the present disclosure, in a specific device, after the excitation light passes through the optical path assembly of the dichroscope and the objective lens, the sample to be tested is excited to generate the fluorescence signal, and the fluorescence signal is subjected to a series of reflections, transmissions or distinguishments in the optical path composed of the optical path assembly of the dichroscope and the objective lens, and is finally collected and imaged by the imaging assembly arranged at a tail end of the optical path, so as to achieve the purpose of detection.

In the method, a principle lies in that at least one type of excitation light irradiates and excites the sample to be tested containing the fluorescent dye to generate the fluorescence signal, and in the excitation light, the maximum wavelength is smaller than the wavelength of the red light; and because the wavelength of the excitation light emitted by the light source assembly is shorter, according to Rayleigh criterion, the resolution is x=0.61 λ/NA, and on the premise that the NA is unchanged, by the preferred excitation light source (blue light) and the matched fluorescent dye, under the specific optical path system of the whole device, the resolution of the optical system is increased by using the blue light with the shorter wavelength without increasing the material cost, thus further improving the sequencing quality or the sequencing throughput, and reducing the sequencing cost.

### BRIEF DESCRIPTION OF DRAWINGS

In order to illustrate the technical schemes in the specific embodiments of the present disclosure or the existing technology more clearly, the drawings which need to be used in describing the specific embodiments or the existing technology will be briefly introduced hereinafter. Apparently, the drawings described hereinafter are only some embodiments of the present disclosure, and those of ordinary skills in the art may further obtain other drawings according to these drawings without going through any creative work.
FIG. 1 is a schematic diagram of a principle of a sequencing device provided by an embodiment of the present disclosure;
FIG. 2 to FIG. 3 are respectively diagrams from two different perspectives of the sequencing device provided by the embodiment of the present disclosure;
FIG. 4 is a front view of FIG. 2 and FIG. 3;
FIG. 5 is a cross-sectional view in a B-B direction of FIG. 4;
FIG. 6 is a diagram from a bottom perspective of the sequencing device provided by the embodiment of the present disclosure;
FIG. 7 is a transmission spectrogram of a dichroscope provided by the embodiment of the present disclosure;
FIG. 8 is a spectrogram of two dyes and corresponding optical filters;
FIG. 9 shows emission intensities of different dyes tested by a microplate reader in the embodiment of the present disclosure;
FIG. 10 shows analysis results of a scatter diagram of blue-green dual-channel sequencing on a sequencing and imaging system of a blue dye and a green dye in two schemes in implementation of the present disclosure; and
FIG. 11 shows SE50 sequencing results based on blue light in implementation of the present disclosure.

In the above drawings, the components represented by the reference numerals are listed as follows:
101 refers to light source assembly; 102 refers to optical path assembly; 103 refers to imaging assembly; and 104 refers to sample to be tested;
201 refers to objective lens; 202 refers to automatic focusing component; 203 refers to second dichroscope; 204 refers to first dichroscope; 205 refers to third dichroscope; 206 refers to cylindrical lens; 207 refers to camera; 208 refers to mounting frame; 209 refers to reflecting component; 210 refers to collimating lens; 211 refers to mounting base; and 213 refers to data interface.

### DETAILED DESCRIPTION

To make the above and other features and advantages of the present disclosure clearer, the present disclosure is further described in detail hereinafter with reference to the drawings. It should be understood that specific embodiments given herein are for the purpose of explanation to those skilled in the art, and are only illustrative and not restrictive.

In the description of the present disclosure, it should be understood that the orientation or position relationship indicated by the terms "center", "longitudinal", "transverse", "length", "width", "thickness", "up", "down", "front", "rear", "left", "right", "vertical", "horizontal", "top", "bottom", "inside", "outside", "clockwise", "anticlockwise", "axial", "radial", "circumferential", and the like is based on the orientation or position relationship shown in the drawings, it is only for the convenience of description of the present disclosure and simplification of the description, and it is not to indicate or imply that the indicated device or element must have a specific orientation, and be constructed and operated in a specific orientation. Therefore, the terms should not be understood as limiting the present disclosure.

Moreover, the terms "first" and "second" are only used for descriptive purposes, but cannot be understood as indicating or implying relative importance, or implicitly indicating the number of indicated technical features. Thus, the feature defined by "first" and "second" may explicitly or implicitly include at least one feature. In the description of the present disclosure, the meaning of "multiple" is at least two, such as two, three, and so on, unless otherwise specifically defined.

In the present disclosure, the terms "installation", "connected", "connection", "fixation", and the like should be understood in broad sense unless otherwise specified and defined. For example, they may be fixed connection, removable connection or integrated connection; may be mechanical connection or electrical connection; and may be direct connection, or indirect connection through an intermediate medium, and connection inside two components, or interaction relation of two elements, unless otherwise specified. The specific meanings of the above terms in the present disclosure may be understood in a specific case by those of ordinary skills in the art.

In the present disclosure, unless otherwise specified and limited, the first feature "on" or "under" the second feature may be direct contact between the first feature and the second feature, and may also be indirect contact between the first feature and the second feature through an intermediate medium. Furthermore, the first feature "on", "above" and "over" the second feature refers to that the first feature is directly above and obliquely above the second feature, or only indicates that the first feature is higher than the second feature in a horizontal height. The first feature "under", "below" and "underneath" the second feature may refer to that the first feature is directly below and obliquely below the second feature, or only indicates that the first feature is lower than the second feature in a horizontal height.

In the descriptions of the specification, the descriptions with reference to the terms "one embodiment", "some embodiments", "example", "specific example" or "some examples", etc., refer to that specific features, structures, materials or characteristics described with reference to the embodiments or examples are included in at least one embodiment or example of the present disclosure. In the specification, the schematic representation of the above terms does not necessarily refer to the same embodiment or example. Moreover, the specific features, structures, materials or characteristics described may be combined in any one or more embodiments or examples in a suitable manner. In addition, in the case of having no mutual contradiction, those skilled in the art may join and combine different embodiments or examples described in the specification and the characteristics of the different embodiments or examples.

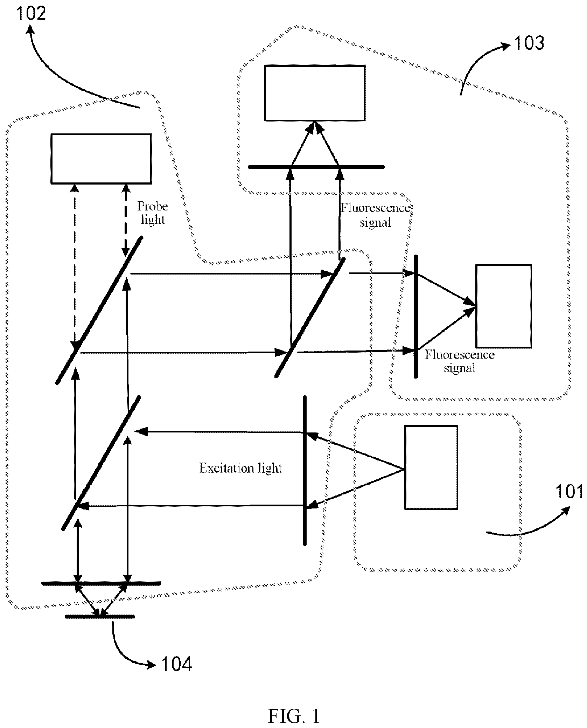

With reference to FIG. 1, one embodiment of a sequencing device provided by the present disclosure is shown, where an arrow direction indicates a forward direction of light. The sequencing device is provided with an excitation light source assembly 101 capable of emitting excitation light with a maximum wavelength smaller than a wavelength of red light, an optical path assembly 102 at least including a dichroscope and an objective lens, and an imaging assembly 103; the dichroscope is arranged on an optical path formed after the excitation light is emitted by the excitation light source assembly 101 for reflection, transmission or distinguishment of the excitation light and/or a fluorescence signal generated after a sample to be tested 104 is excited by the excitation light; and the objective lens is arranged on an optical path through which the fluorescence signal enters the dichroscope; and the imaging assembly 103 is arranged on an optical path formed after the fluorescence signal is distinguished for collecting and imaging different fluorescence signals.

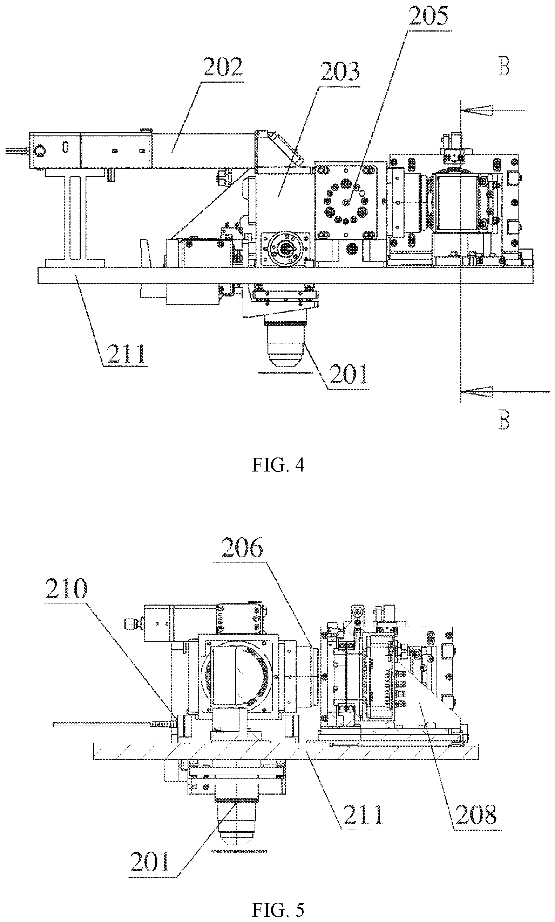

With reference to FIG. 1 to FIG. 11, the preferred embodiment of the present disclosure may be a combination with any one or more of the followings on the basis of the above embodiment.

The excitation light source assembly includes two excitation light sources, where one excitation light source excites blue light as a first excitation light, and the other excitation light source excites green light as a second excitation light; and the two excitation light sources are coupled through optical fibers, and the two types of excitation light share one optical fiber port for output. An automatic focusing component 202 is arranged in the optical path assembly; the automatic focusing component 202 is capable of emitting probe light with a wavelength different from the wavelength of the fluorescence signal; and the probe light is transmitted through the dichroscope and the objective lens 201, and then irradiated to the sample to be tested, and then returns in the same path, and the automatic focusing component 202 drives the objective lens 201 to realize focusing according to a feedback signal collected after returning.

The dichroscope includes a first dichroscope 204, a second dichroscope 203 and a third dichroscope 205, where the second dichroscope 203 and the first dichroscope 204 are arranged from top to bottom; and the third dichroscope 205 and the second dichroscope 203 are communicated through an optical path and arranged adjacently.

The imaging assembly includes a cylindrical lens 206 and a camera 207 which are arranged on different output optical paths of the third dichroscope 205; and a reflecting component 209 is also arranged between the cylindrical lens 206 and the camera 207.

A collimating lens 210 is arranged on an optical path between the excitation light source assembly and the first dichroscope 204; a free end of the collimating lens 210 is used for accessing the excitation light emitted by the excitation light source assembly; and the other end of the collimating lens extends inside the first dichroscope 204. The excitation light is collimated by the collimating lens 210, then reflected by the first dichroscope 204 to the objective lens 201, and then focused on the sample to be tested.

The dichroscope, the imaging assembly and the focusing component are all arranged on a mounting base 211; one end of the objective lens 201 penetrates through the mounting base 211 to be opposite to the sample to be tested, and the other end of the objective lens is communicated with the first dichroscope 204; and data interfaces 213 for data exchange are preset on the camera 207 and the automatic focusing component 202.

A filtering component may also be arranged in an optical path in front of or behind the cylindrical lens 206 to realize a filtering effect, and the arrangement of the filtering component in the optical path in front of or behind the cylindrical lens 206 is not strictly limited, which is generally selected according to actual operation and convenience of device.

In some schemes, the filtering component is arranged between the camera 207 and the cylindrical lens 206, and in some embodiments, the filtering component is arranged between the third dichroscope 205 and the cylindrical lens 206; the objective lens 201 is provided with a plurality of optical lenses, and the objective lens 201 is a dry or immersive objective lens, where the immersive objective lens may also be a water immersive or oil immersive objective lens; and a magnification factor of the objective lens 201 is not less than 16 times.

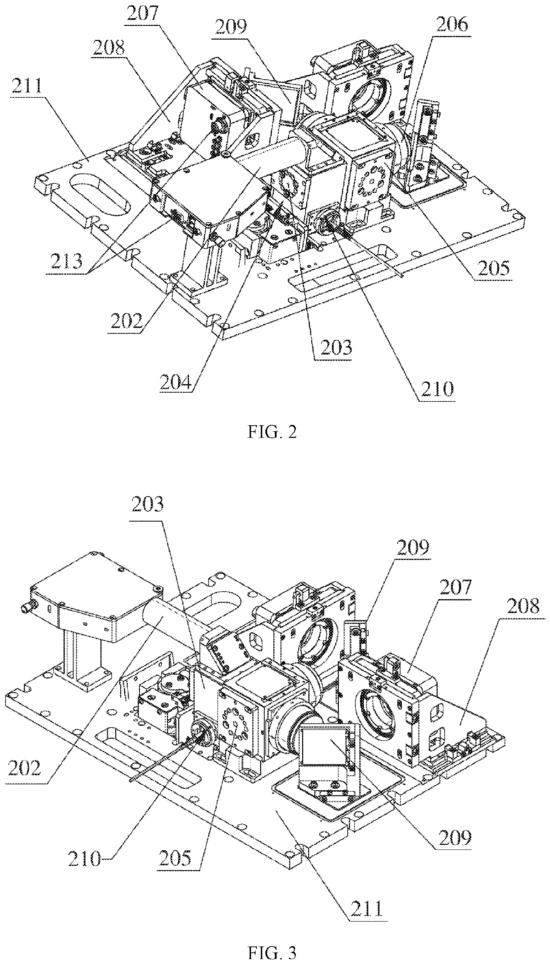

With reference to FIG. 2 to FIG. 11, in some embodiments, the sequencing device provided by the present disclosure includes the light source, the collimating lens 210, the first dichroscope 204, the second dichroscope 203, the third dichroscope 205, the objective lens 201, the automatic focusing component 202, the imaging assembly, and the like, where the imaging assembly includes the cylindrical lens 206 and the camera 207 arranged on different output optical paths of the third dichroscope 205, and the reflecting component 209 is arranged between the cylindrical lens 206 and the camera 207. The collimating lens 210, the first dichroscope 204, the second dichroscope 203, the third dichroscope 205, the automatic focusing component 202 and the imaging assembly are all arranged on the mounting base 211, the second dichroscope 203 and the third dichroscope 205 are communicated through the optical path, and the cylindrical lens 206 and the camera 207 are both arranged in a pair for collecting and imaging the two fluorescence signals.

In other schemes, the filtering component may also be arranged between the cylindrical lens 206 and the reflecting component 209, and in addition, in some embodiments, an optical filter is directly used as the filtering component.

More specifically, the light source (not shown in the figures) contains two types of excitation light, one type of excitation light comes from a solid-state semiconductor (DPSS) laser device of 473 nm; and the other type of excitation light comes from a solid-state semiconductor (DPSS) laser device of 561 nm. The two types of excitation light are coupled through the optical fibers inside the light source, and then output from the same optical fiber port, and the optical fiber port is an FC/APC interface for reducing a return loss.

Example parameters of the light source refer to Table 1 below.

**Table 1 Parameters of light source**

| **Name of material** | **Typical value** |
|---|---|
| Output power of optical fiber (mw) | 473 nm≥2000 |
| | 561 nm≥2000 |
| Center wavelength (nm) | 473±3 |
| | 561±3 |
| Uniformity of light spots | >85% |
| Model of output optical fiber | Core diameter 400 µm 0.22 NA |
| Working temperature (°C) | 20-40 |
| Laser head | FC/APC |
| Length of optical fiber (mm) | >2000 |

The excitation light is collimated by the collimating lens 210, guided into the objective lens 201 by the first dichroscope 204, and finally projected onto a surface of the sample to be tested. A focal length of the collimating lens 210 is adjusted according to a required illumination range, and in the embodiment, the focal length is 2.9 mm.

The first dichroscope 204 may distinguish the excitation light from the fluorescence signal, where the first dichroscope 204 guides the excitation light into the objective lens 201 while allowing the fluorescence signal sent from the sample to be tested to transmit.

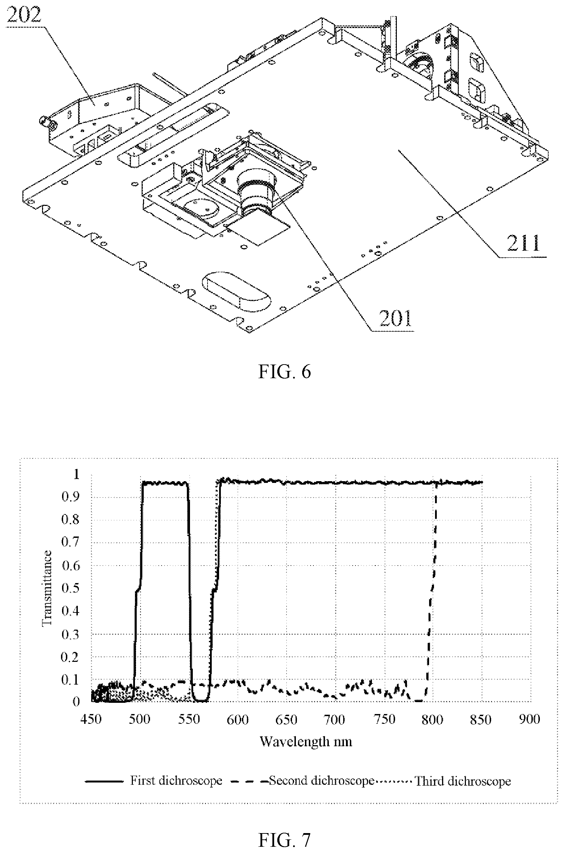

FIG. 7 shows a spectrum of the dichroscope, in which an abscissa represents a wavelength and an ordinate represents a transmittance. In the embodiment, a reflectivity of the first dichroscope 204 to an excitation light spectrum of 473/561 nm is above 95%, and a transmittance to fluorescent wavebands of 500 nm to 550 nm and 580 nm to 650 nm is above 95%.

The objective lens 201 receives the excitation light reflected from the first dichroscope 204 to project the excitation light onto the sample to be tested. In the embodiment, the objective lens 201 includes the plurality of optical lenses, with the magnification factor of 16X and the NA of 0.8.

In one embodiment, the sample to be tested is a biological sample, which contains a DNA sequence and a fluorescent dye, and the fluorescent dye may bind to the DNA through some binding enzymes; and the DNA is arranged on a substrate, and the substrate includes one of a silicon substrate, glass or sapphire, and is preferably the silicon substrate. Two fluorescent dyes above are provided, where one fluorescent dye is excited by blue light, which is called a first fluorescent dye, with a sent fluorescence signal called a first fluorescence signal; and the other fluorescent dye is excited by green light, which is called a second fluorescent dye, with a sent fluorescence signal called a second fluorescence signal.

The selection of the fluorescent dye corresponds to the wavelength of the excitation light, so as to improve excitation efficiency as much as possible. In the embodiment, Alexa Fluor 488 and ROX are taken as the first and second fluorescent dyes respectively.

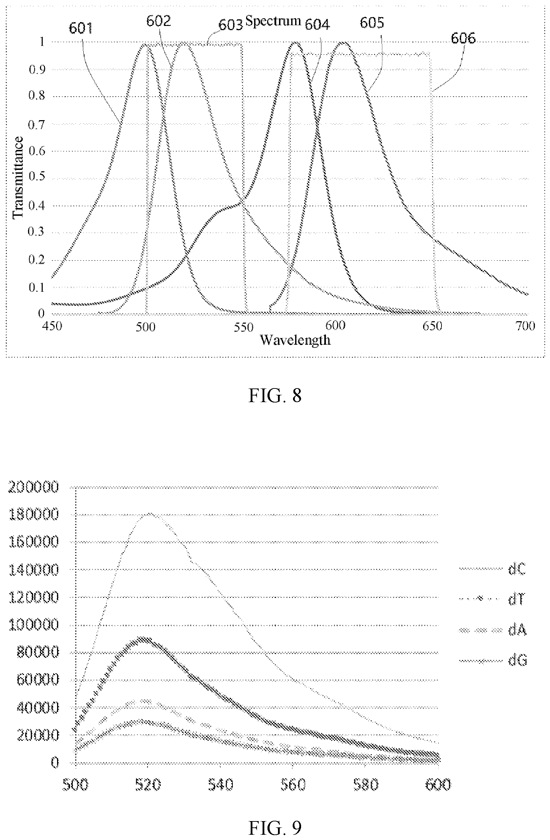

FIG. 8 is a spectrogram of the two dyes above and corresponding filtering components (the filter components are specifically set as optical filters and arranged in front of the camera 207, specifically between the camera 207 and the cylindrical lens 206, and are not shown in the figure). In FIG. 8, excitation efficiency of an absorption spectrum 601 of the first fluorescent dye Alexa Fluor 488 at a first excitation wavelength of 473 nm is about 0.4, a transmission window of a first optical filter 603 is 500 nm to 550 nm, and combination efficiency of an emission spectrum 602 of the first fluorescent dye and the first optical filter 603 is about 0.67.

Excitation efficiency of an absorption spectrum 604 of the second fluorescent dye ROX at 561 nm is about 0.59, a transmission window of a second optical filter 606 is 580 nm to 650 nm, and combination efficiency of an emission spectrum 605 of the second fluorescent dye and the second optical filter 606 is about 0.59. The arrangement of the optical filter in the embodiment significantly filters the excitation light and allows the fluorescence signal to transmit, thus achieving a good distinguishing effect.

It should be noted that a part of the emission spectrum 602 of the first fluorescent dye above coincides with a spectrum of the second optical filter 606, and this part of the signal is called Crosstalk. Crosstalk is actually equivalent to background noise for a camera acquisition system, which may affect a signal-to-noise ratio of the system, and in a process of selecting fluorescent dye combinations, the combinations with less Crosstalk should be selected as far as possible. In the embodiment, through the combination of the fluorescent dye and the optical filter, Crosstalk is about 8%, which reaches a higher signal-to-noise ratio, thus making the sequencing quality more accurate.

The two fluorescence signals from the sample to be tested are collected by the objective lens 201 and allowed to transmit through the first dichroscope 204 to reach the second dichroscope 203. The second dichroscope 203 distinguishes the fluorescence signal from a probe signal of the automatic focusing component 202. in the embodiment, the second dichroscope 203 is a long-pass optical filter of 800 nm. The fluorescence signal ranges from 450 nm to 750 nm.

The probe light emitted by the automatic focusing component 202 is infrared light, which has a wavelength different from the wavelength of the fluorescence signal. Specifically, a waveband of the probe signal of the automatic focusing component 202 is 800 nm to 900 nm, and preferably 820 nm to 860 nm. the probe light emitted by the automatic focusing component 202 may pass through the second dichroscope 203 and the first dichroscope 204 in sequence to reach the sample to be tested, and then return to the automatic focusing component 202 from the sample to be tested in the same path. The automatic focusing component 202 drives the objective lens 201 to move to a focal plane position according to the collected feedback signal to realize the focusing function.

Further, the fluorescence signal is reflected from the second dichroscope 203 to the third dichroscope 205, the third dichroscope 205 may separate the first fluorescence signal from the second fluorescence signal, and guide the signals into two cameras 207 respectively. A spectrum of the third dichroscope 205 is shown in FIG. 7. The first fluorescence signal and the second fluorescence signal excited by the blue light and the green light are reflected from the third dichroscope 205 to the cylindrical lens 206, and finally collected by the camera 207.

In one preferred embodiment of the present disclosure, the dichroscope includes the first dichroscope 204 and the second dichroscope 203, and the second dichroscope 203 and the first dichroscope 204 are arranged from top to bottom. The two dichroscopes both have a fine-tuning structure to ensure that the dichroscopes are located at appropriate angles.

The objective lens 201 is fixed on an electric displacement table in a threaded manner, and the objective lens 201 is locked on a focal plane of the sample to be tested by combining with the automatic focusing component 202. The electric displacement table is driven by a voice coil motor to ensure a requirement of high accuracy. Specifically, a positioning accuracy is better than 100 nm, and preferably, the accuracy is better than 20 nm.

The DNA (globular) and the fluorescent dye are attached to the surface of the sample to be tested. The sample to be tested is placed on a two-dimensional electric translation table, and under the cooperation of the automatic focusing component 202 and the two-dimensional electric translation table, the sample to be tested may be fully scanned and observed. Optionally, the two-dimensional electric translation table is driven by a linear motor, so that requirements of long scanning stroke and high-accuracy positioning are met. In the embodiment, a stroke range is greater than 80 mm*80 mm, and a positioning accuracy is better than 200 nm.

The fluorescence signal may pass through the cylindrical lens 206 and the reflecting component to enter the camera 207. The reflecting component 209 is used for folding the optical path to make a structure more compact, and the reflecting component 209 has a two-dimensional rotationally adjustable structure to ensure the alignment of the optical path.

The cylindrical lens 206 is mounted on a base on which the third dichroscope 205 is located by a mounting mode of threaded fixing, sleeved fixing, and the like. In order to eliminate an influence of ambient light, a lens hood (not shown in the figure) is also arranged in a space between the cylindrical lens 206 and the camera 207.

The camera 207 is mounted on a base with five-axis adjustment, and integrated with the mounting base 211 through a mounting frame 208. According to actual incident angle and position of the fluorescence signal, the camera 207 may be adjusted, so that a target surface of the camera 207 is aligned with an incident light path. More specifically, the camera 207 is a CMOS detector, with a pixel number better than 5000*5000 pixels.

In other embodiments of the present disclosure, in order to eliminate chromatic aberration, the collimating lens 210 may be a cemented doublet. In addition, the objective lens 201 is the immersive objective lens, which may be the water immersive or oil immersive objective lens. For blue light with a wavelength of 473 nm, selectable first fluorescent dyes further include iF488, ATTO488, DyLight488, Hilyte488, FAM488 or FITC488, and the like; and for green light with a wavelength of 561 nm, selectable second fluorescent dyes include CY3.5, AF568, and the like.

In other preferred embodiments of the present disclosure, efficiency of the system can be improved by customizing a spectral line of the optical filter. For example, the window of the first optical filter 603 can be optimized to 483 nm to 550 nm, and the window of the second optical filter 606 can be optimized to 580 nm to 700 nm. Meanwhile, Crosstalk may be less than 5% by selecting different dye combinations and optimizing the spectrum of the optical filter. Because Crosstalk is very low, there is a very high signal-to-noise ratio, so that the sequencing quality is accurate.

In some preferred embodiments of the present disclosure, the waveband of the first fluorescence signal is 500 nm to 550 nm, and the waveband of the second fluorescence signal band is 580 nm to 650 nm. In addition, for the two-dimensional electric translation table, the stroke range is preferably greater than 100* 100 mm. In a more preferred embodiment, the positioning accuracy is better than 50 nm.

A more complex sequencing system may be formed by adding the sample to be tested including the DNA sequence and the fluorescent dye to the sequencing device mentioned in any of the schemes above and making the sample to be tested opposite to the objective lens. The fluorescent dye is excited by the excitation light emitted by the excitation light source assembly to generate the fluorescence signal, and the fluorescence signal enters the optical path of the dichroscope through the objective lens to realize the sequencing effect.

In a preferred embodiment, a control component is included, and the control component is connected with one or more assemblies of the sequencing device for data exchange, control or display.

The embodiment of the present disclosure further provides a method for increasing a sequencing resolution, which includes the following steps of:
carrying out irradiation and excitation on a sample to be tested containing a fluorescent dye with at least one type of excitation light to generate a fluorescence signal, where a maximum wavelength in the excitation light is smaller than a wavelength of red light; and
transmitting the generated fluorescence signal through an optical path, and collecting and imaging the signal.

In some specific schemes, the blue light with the wavelength ranging from 440 nm to 500 nm is used as the first excitation light; and the green light with the wavelength ranging from 500 nm to 600 nm is used as the second excitation light. The two types of excitation light are coupled through the optical fibers and share one optical fiber port for output and transmission to the sample to be tested, and a type of the optical fiber port is one of FC/PC or FC/APC or SMA.

In a more specific scheme, the wavelength of the first excitation light ranges from 470 nm to 490 nm; and the wavelength of the second excitation light ranges from 550 nm to 580 nm. Uniformities of light spots of the two types of excitation light are both greater than 85%; lengths of optical fibers of the two types of excitation light are both greater than 2,000 mm; and output power of the optical fibers is greater than or equal to 2,000 mw. In a more preferred scheme, the first excitation light is the blue light, with the wavelength of 470 nm, 473 nm or 488 nm; and the second excitation light is the green light, with the wavelength of 556 nm, 561 nm or 577 nm.

It should be noted that, according to an analysis need, the wavelength of the blue light may be extended to the green light, and the wavelength of the green light may be extended to yellow and red light.

In other specific schemes, the first excitation light is the blue light, with the wavelength simply having an upper limit, such as 470 nm, 473 nm or 488 nm; and the second excitation light is the green light, with the wavelength simply having a lower limit, such as 556 nm, 561 nm or 577 nm.

Based on the sequencing device mentioned above, in combination with the above method, another specific implementation method provided by the present disclosure, in which two types of excitation light with different wavelengths which are less than a wavelength of red light are emitted by the excitation light source assembly; the excitation light is reflected by the first dichroscope 204, passes through the objective lens 201, and is irradiated to the sample to be tested (containing two fluorescent dyes), and two fluorescence signals are generated; the two fluorescence signals are allowed to transmit the first dichroscope 204, reflected by the second dichroscope 203, distinguished by the third dichroscope 205, and then collected and imaged by the imaging assembly.

Corresponding to the above device, in some embodiments, the transmitting through the optical path further includes the step of respectively guiding the fluorescence signal and a non-fluorescence signal to different optical paths by using two types of dichroscopes; where a transmission window of one type of dichroscope is 500 nm to 550 nm and/or 580 nm to 650 nm; and a transmission window of the other type of dichroscope is at least 800 nm.

In some embodiments in combination with the device above, the transmitting the fluorescence signal through the optical path further includes the operation of making the signal pass through a filtering component with a transmission window of 483 nm to 550 nm or 580 nm to 700 nm.

In a preferred embodiment of the present disclosure, for the fluorescent dye, test optimization is carried out. Specifically, according to the light source and the optical filter, fluorescent dyes with matching absorption and emission wavelengths are screened. For the first excitation light (blue light), available dyes include Alexa Fluor 488, iF488, ATTO488, DyLight488, Hilyte488, FAM488, FITC488, and the like, hereinafter referred to as a first dye; and for the second excitation light (green light), AF568, ROX dye in DNBSEQTM technology, and the like may be selected, hereinafter referred to as a second dye.

Taking Alexa Fluor 488 as an example, basic groups are labeled with the first dye. Four basic groups A, C, G and T are respectively labeled with the selected first dye, and then a dye performance is tested by a microplate reader.

FIG. 9 shows emission intensities of different dyes tested by the microplate reader. Results show that, due to physical and chemical properties of the fluorescent dye, yields of labeled products are different and luminous intensities are also different in a labeling reaction. When the basic groups G and A are labeled with Alexa Fluor 488, there is the worst luminescence effect. In a cPAS scheme of blue light, the basic groups C and T are labeled with the first dye, and the basic groups A and G are labeled with the second dye.

Further preferably, the first dye is Alexa Fluor 488; and the second dye is ROX.

It should be understood that cPAS is a well-known technical term in the art, which is short for Combined Probe Anchor Synthesis (cPAS).

In a dual-color sequencing scheme, basic group labeling schemes of dyes may be combined in different ways according to actual needs, for example, the basic group C may be labeled with two dyes, or the basic group T may be labeled with two dyes, as shown in the following table 2:

**Table 2 Labeling schemes of dyes**

| **First scheme** | **Second scheme** |
|---|---|
| A-second dye | A-second dye |
| C-second dye | C-first dye |
| C-first dye | T-second dye |
| T-first dye | T-first dye |
| G-no label | G-no label |

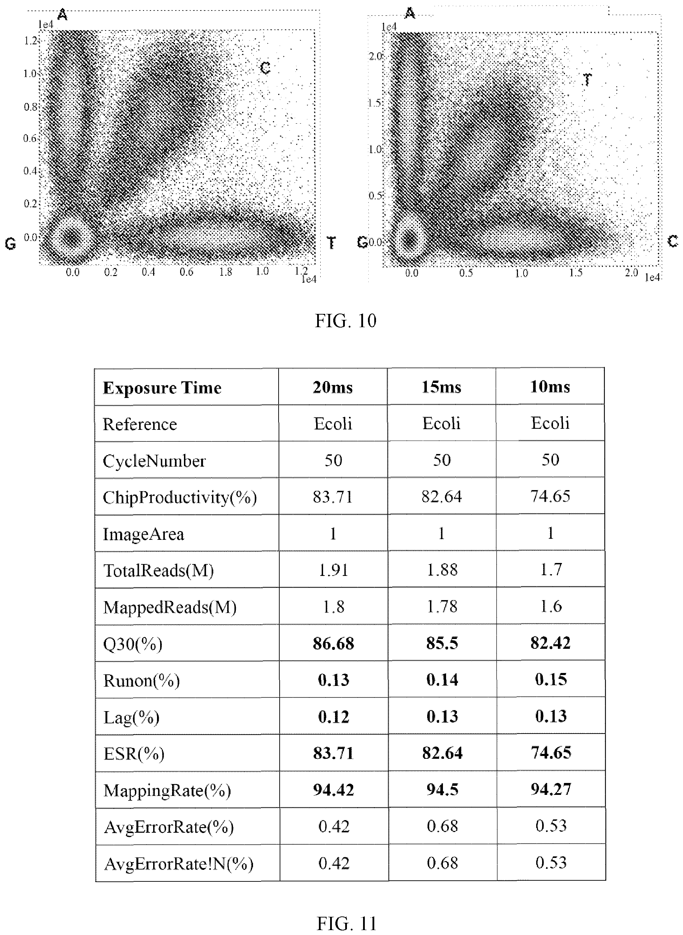

FIG. 10 shows analysis results of a scatter diagram of blue-green dual-channel sequencing on a sequencing and imaging system of the first dye and the second dye in two schemes, where (a) shows the analysis result of the scatter diagram of blue-green dual-channel sequencing on the sequencing and imaging system of the first dye and the second dye in the first scheme; and (b) shows the analysis result of the scatter diagram of blue-green dual-channel sequencing on the sequencing and imaging system of the first dye and the second dye in the second scheme. An abscissa represents a blue light channel, and an ordinate represents a green light channel.

It can be seen from FIG. 10 that scatter results of blue-green dual-channel sequencing on the sequencing and imaging system of the first dye and the second dye in the two schemes all show that the four basic groups are less connected in series with each other, with obvious distinguishing effect.

Further, an optimization test is carried out on proportions of the two dyes used for labeling the basic groups in the cPAS scheme above. Specifically, the labeling mode of the first scheme in Table 2 above is used, the two dyes are used for labeling different basic groups, and different dosage proportions of the first dye and the second dye are set. The proportions and initial Q30 in the test are shown in Table 3 below:

**Table 3 Statistical table of proportions and initial Q30 in test**

| | **1** | **2** | **3** | **4** | **5** | **6** | **7** | **8** | **9** |
|---|---|---|---|---|---|---|---|---|---|
| **First dye** | 50% | 60% | 65% | 70% | 80% | 78% | 75% | 75% | 75% |
| **Second dye** | 50% | 40% | 35% | 30% | 20% | 22% | 25% | 25% | 25% |
| **Q30 (%)** | 57.4 | 59.8 | 62.1 | 68.0 | 70.3 | 73.1 | 80.9 | 81.7 | 84.74 |

In this experiment, a total dosage of the first dye and the second dye is 1µmol, and combinations 1 to 9 show conditions of different dosages of the first dye and the second dye respectively. For example, according to first proportions, dosages of the first dye and the second dye are 0.5µmol respectively, and specific dosages of the first dye and the second dye in other combinations are determined according to the proportions, which will not be repeated herein.

According to results of initial Q30, a range of a dosage ratio of the first dye to the second dye is fixed between 3 and 4. According to test results, when a proportion combination that the first dye/the second dye=3 is selected, an SE50 test is carried out on the sequencing device in the above preferred embodiment.

Results are shown in FIG. 11, a mapping rate of SE50 sequencing level based on the blue light is 94% to 95%, and under exposure of 15 ms and 20 ms, the Q30 meets the sequencing requirement.

To sum up, in the present disclosure, under the premise that the NA is unchanged, by setting a specific optical path structure for the sequencing device and selecting specific excitation light sources (blue light and green light) and matched fluorescent dyes, the resolution of the optical system is increased by using a blue-green light excitation mode with a shorter wavelength (the first excitation light is the blue light and the second excitation light is the green light) without increasing the material cost, thus improving the sequencing quality or the sequencing throughput, and reducing the sequencing cost.

Although the embodiments of the present disclosure have been shown and described above, it may be understood that the above embodiments are exemplary and cannot be understood as limiting the present disclosure, and those of ordinary skills in the art may make changes, modifications, substitutions and variations to the above embodiments within the scope of the present disclosure.

## Claims

1. A method for increasing a sequencing resolution, comprising the following steps of:
carrying out irradiation and excitation on a sample to be tested containing a fluorescent dye with at least one type of excitation light to generate a fluorescence signal, wherein a maximum wavelength in the excitation light is smaller than a wavelength of red light; and
transmitting the generated fluorescence signal through an optical path, and collecting and imaging the signal.

2. The method for increasing the sequencing resolution according to claim 1, wherein the excitation light takes blue light as first excitation light and takes green light as second excitation light.

3. The method for increasing the sequencing resolution according to claim 2, wherein a wavelength of the first excitation light ranges from 440 nm to 500 nm, and a wavelength of the second excitation light ranges from 500 nm to 600 nm.

4. The method for increasing the sequencing resolution according to claim 2, wherein a wavelength of the first excitation light ranges from 470 nm to 490 nm, and a wavelength of the second excitation light ranges from 550 nm to 580 nm.

5. The method for increasing the sequencing resolution according to claim 2, wherein uniformities of light spots of the two types of excitation light are both greater than 85%; lengths of optical fibers of the two types of excitation light are both greater than 2,000 mm; and output power of the optical fibers is greater than or equal to 2,000 mw.

6. The method for increasing the sequencing resolution according to claim 2, wherein the fluorescent dye comprises a first fluorescent dye excited by the first excitation light and a second fluorescent dye excited by the second excitation light;
the first fluorescent dye comprises: one or more of Alexa Fluor 488, iF488, ATTO488, DyLight488, Hilyte488, FAM488 or FITC488; and
the second fluorescent dye comprises: one or more of CY3.5, AF568 or ROX.

7. The method for increasing the sequencing resolution according to claim 6, wherein a dosage ratio of the first fluorescent dye to the second fluorescent dye is 3: 1 to 4: 1.

8. The method for increasing the sequencing resolution according to claim 2, wherein the transmitting through the optical path further comprises the step of respectively guiding the fluorescence signal and a non-fluorescence signal to different optical paths by using two types of dichroscopes; wherein a transmission window of one type of dichroscope is 500 nm to 550 nm and/or 580 nm to 650 nm; and a transmission window of the other type of dichroscope is at least 800 nm.

9. The method for increasing the sequencing resolution according to claim 2, wherein the transmitting the fluorescence signal through the optical path further comprises the operation of making the signal pass through a filtering component with a transmission window of 483 nm to 550 nm or 580 nm to 700 nm.

10. The method for increasing the sequencing resolution according to any one of claims 1 to 9, wherein the sample to be tested comprises DNA and is arranged on a substrate, and the substrate comprises one of a silicon substrate, glass or sapphire.

11. A sequencing device for implementing the method according to any one of claims 1 to 10, wherein the sequencing device is provided with an excitation light source assembly capable of emitting the excitation light with the maximum wavelength smaller than the wavelength of the red light, an optical path assembly at least comprising a dichroscope and an objective lens, and an imaging assembly;
the dichroscope is arranged on an optical path formed after the excitation light is emitted by the excitation light source assembly for reflection, transmission or distinguishment of the excitation light and/or the fluorescence signal generated after the sample to be tested is excited by the excitation light; and the objective lens is arranged on an optical path through which the fluorescence signal enters the dichroscope; and
the imaging assembly is arranged on an optical path formed after the fluorescence signal is distinguished for collecting and imaging different fluorescence signals.

12. The sequencing device according to claim 11, wherein the excitation light source assembly comprises two excitation light sources, wherein one excitation light source excites the blue light as the first excitation light, and the other excitation light source excites the green light as the second excitation light; and
the two excitation light sources are coupled through the optical fibers, and the two types of excitation light share one optical fiber port for output.

13. The sequencing device according to claim 11, wherein the optical path assembly further comprises an automatic focusing component; the automatic focusing component emits probe light with a wavelength different from the wavelength of the fluorescence signal; and the probe light is transmitted through the dichroscope, the objective lens and the sample to be tested and returns in the same path, and the automatic focusing component drives the objective lens to realize focusing according to a feedback signal collected after returning.

14. The sequencing device according to any one of claims 11 to 13, wherein the dichroscope comprises a second dichroscope and a first dichroscope arranged from top to bottom; and
a third dichroscope, wherein the third dichroscope and the second dichroscope are communicated through an optical path and arranged adjacently.

15. The sequencing device according to claim 14, wherein the imaging assembly comprises a cylindrical lens and a camera which are arranged on different output optical paths of the third dichroscope; and
a reflecting component is also arranged between the cylindrical lens and the camera.

16. The sequencing device according to claim 14, wherein a collimating lens is arranged on an optical path between the excitation light source assembly and the first dichroscope; a free end of the collimating lens is used for accessing the excitation light emitted by the excitation light source assembly; and the other end of the collimating lens extends inside the first dichroscope.

17. The sequencing device according to claim 14, further comprising a mounting base, wherein the dichroscope and the imaging assembly are arranged on the mounting base;
and/or; in response to the optical path assembly comprising the automatic focusing component, the automatic focusing component is arranged on the mounting base; and
one end of the objective lens penetrates through the mounting base and is used for being opposite to the sample to be tested, and the other end of the objective lens is communicated with the first dichroscope and/or the second dichroscope.

18. The sequencing device according to claim 15, wherein a data interface for data exchange is preset on the camera;
and/or; in response to the optical path assembly comprising the automatic focusing component, a data interface for data exchange is preset on the automatic focusing component.

19. The sequencing device according to claim 15, further comprising a filtering component, wherein the filtering component is arranged in an optical path in front of or behind the cylindrical lens.

20. The sequencing device according to claim 19, wherein the filtering component is arranged between the third dichroscope and the cylindrical lens.

21. The sequencing device according to claim 14, wherein the objective lens is provided with a plurality of optical lenses, and the objective lens is a dry or immersive objective lens, with a magnification factor not less than 16 times.

22. A sequencing system, comprising a sample to be tested and the sequencing device according to any one of claims 11 to 21, wherein,
the sample to be tested comprises a DNA sequence and a fluorescent dye, and is opposite to the objective lens; and
in response to the fluorescent dye being excited by the excitation light emitted by the excitation light source assembly, the fluorescence signal is generated and enters the optical path of the dichroscope through the objective lens.

23. The sequencing system according to claim 22, further comprising a control component, wherein the control component is connected with one or more assemblies of the sequencing device for data exchange, control or display.
